# FASCICULE DE BREVET EUROPEEN

(11) **EP 2 986 591 B1**
(45) Date de publication et mention de la délivrance du brevet: **05.04.2017**
(21) Numéro de dépôt: 14725224.1
(22) Date de dépôt: 16.04.2014
(51) Int. Cl.: C07C 253/26

(54) **PROCEDE DE PRODUCTION DE NITRILES METTANT EN UVRE UN CATALYSEUR A BASE D'ANTIMOINE ET DE FER**
VERFAHREN ZUR HERSTELLUNG VON NITRILEN UNTER VERWENDUNG EINES KATALYSATORS AUS ANTIMON UND EISEN
METHOD FOR PRODUCING NITRILES USING A CATALYST MADE FROM ANTIMONY AND IRON

(30) Priorité: 17.04.2013 FR 1353478
(43) Date de publication de la demande: 24.02.2016
(73) Titulaire: Centre National de la Recherche Scientifique (C.N.R.S.), 75016 Paris (FR); Ecole Centrale De Lille, 59651 Villeneuve d'Ascq (FR); Université des Sciences et Technologies De Lille - Lille 1, 59655 Villeneuve d'Ascq (FR)
(72) Inventeur: PAUL, Sébastien, F-59158 Thun St Amand (FR); KATRYNIOK, Benjamin, F-62750 Loos-en-gohelle (FR); DUMEIGNIL, Franck, F-59273 Fretin (FR); LIEBIG, Carsten, 67549 Worms (DE); HÖLDERICH, Wolfgang, 67227 Franckenthal (DE); GUILLON, Cyrille, F-59000 Lille (FR)
(74) Mandataire: Goulard, Sophie
(86) Numéro de dépôt international: PCT/FR2014/050924
(87) Numéro de publication internationale: WO 2014/170604

(56) Documents cités:
- WO-A1-2009/063120
- ALLEN, M.D. ET AL.: "Two-dimentional oxide catalysts: propene oxidation on FeSbO4", CATALYSIS LETTERS, vol. 33, 1995, pages 269-277, XP002717531,

## Description

La présente invention est relative à l'utilisation d'un catalyseur à base d'antimoine et de fer pour catalyser la production de nitriles, notamment d'acrylonitrile, ainsi qu'à un procédé de production de nitriles, notamment d'acrylonitrile, en phase gaz, en présence d'un tel catalyseur.

Le propènenitrile (CH₂=CH-C≡N), plus communément appelé acrylonitrile, est une matière première utilisable à titre de monomère pour la préparation de nombreux polymères en chimie organique, notamment de fibres textiles comme les fibres acryliques, le nylon, le caoutchouc synthétique, ou bien d'élastomères azotés après copolymérisation avec du butadiène, ou bien encore de résines solides de hautes performances (résines acrylonitrile butadiène styrène : ABS) après copolymérisation avec du butadiène et du styrène.

La production industrielle d'acrylonitrile est aujourd'hui dépendante du propène ou du propane qui sont tous deux des composés issus des ressources fossiles. En effet, la synthèse de l'acrylonitrile se fait habituellement selon le procédé dit « Sohio » (du nom de la société Standard Oil of Ohio), développé dans les années 60, et tel que décrit, par exemple, dans la demande de brevet GB 709 337. Ce procédé consiste à réaliser l'oxydation du propène en présence d'ammoniac (ammoxydation) selon la réaction suivante :

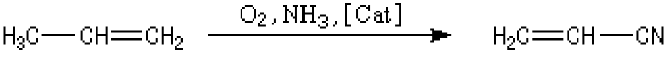

Cette réaction est réalisée en phase gaz dans un réacteur à lit fluidisé, à haute température, en général à 350-400°C, en absence d'eau et en présence d'un catalyseur à base de bismuth et de molybdène.

Cependant, ce procédé présente l'inconvénient de mettre en oeuvre du propène dont le prix de revient ne cesse d'augmenter.

Il existe donc un besoin pour un procédé de préparation d'acrylonitrile, qui soit indépendant du propène, tout en conduisant à d'excellents rendements. Les inventeurs se sont notamment donné pour but de mettre au point un procédé permettant de produire de l'acrylonitrile à partir d'alcool allylique. Par ailleurs, ce dernier peut être biosourcé, ce qui apporte un avantage environnemental à un tel procédé, même si ce dernier ne s'avérait pas, *in fine*, économique. C'est lors de ces recherches que les inventeurs ont découvert que l'utilisation d'un catalyseur particulier à base d'antimoine et de fer permettait notamment de catalyser la réaction de production d'acrylonitrile à partir d'alcool allylique en phase gaz avec un très bon rendement. Le document WO2009/063120 divulgue un procédé de production à partir d'un alcool. La présente invention a donc pour premier objet l'utilisation d'un composé de formule (I) suivante :

SbₓFe₁O_{y} (I)

dans laquelle x varie de 0,4 à 1 inclusivement, et y varie de 1,6 à 4 inclusivement, à titre de catalyseur pour catalyser la réaction d'ammoxydation d'un alcool de formule (II) suivante CH₂=C(R¹)-CH₂-OH (II) dans laquelle R¹ représente un atome d'hydrogène ou un radical méthyle en un nitrile de formule (III) suivante CH₂=C(R¹)-C≡N (III) dans laquelle R¹ a la même signification que dans la formule (II) ci-dessus, ladite réaction étant réalisée en phase gaz, ladite phase gaz comprenant au moins de l'oxygène et de l'ammoniac.

L'utilisation du catalyseur de formule (I) ci-dessus permet de réaliser la réaction d'ammoxydation d'un alcool de formule (II) pour conduire à un nitrile de formule (III), correspondant, en particulier à l'acrylonitrile, en phase gaz avec un très bon rendement. Cette réaction permet également d'accéder, à titre de sous-produits, à l'acroléine (et à l'acide acrylique par oxydation subséquente de l'acroléine), l'acétone, l'acétaldéhyde, l'acétonitrile et au propionaldéhyde, lesquels sont également valorisables car il s'agit d'intermédiaires largement utilisés dans l'industrie chimique. De plus, au vu des différents points d'ébullition de chacun de ces produits (acétaldéhyde : 22°C ; propionaldéhyde : 47°C ; acroléine : 52°C ; acétone 57°C ; acrylonitrile : 77°C, acétonitrile : 82°C ; acide acrylique : 147°C), leur séparation est théoriquement facile à mettre en oeuvre, par exemple au sein même d'un procédé intégré dédié.

De plus, comme évoqué précédemment, la matière première (l'alcool de formule (II)) peut être produite à partir de ressources renouvelables, ce qui permet de s'affranchir des ressources fossiles pour accéder aux nitriles de formule (III), et, en particulier, à l'acrylonitrile. Par exemple, le glycérol, seul ou en mélange avec de l'acide formique, peut être utilisé pour produire de l'alcool allylique selon divers procédés catalytiques en phase gaz ou en phase liquide décrits dans la littérature, notamment dans les demandes internationales WO 2008/092115 et WO 2011/08509.

A titre d'exemple, le catalyseur de formule (I) ci-dessus peut notamment être préparé selon le procédé décrit par Allen M.D. et al. (Catalysis Letters 33, 1995, 269) ou encore par Li K.-T. et al. (Applied Catalysis A : General 156, 1997, 117-130). Brièvement, ce procédé consiste à faire réagir une solution aqueuse acide d'un sel de fer, tel que par exemple le nitrate de fer, à une température de 80°C environ, avec de l'oxyde d'antimoine (Sb₂O₃) puis à maintenir la température entre 80 et 90°C pendant plusieurs heures afin d'évaporer le solvant et obtenir un produit pâteux qui est ensuite séché à environ 100°C pendant plusieurs jours. La poudre de catalyseur obtenue est ensuite pressée sous forme de pastilles qui sont ensuite broyées en une poudre qui est, de préférence, calcinée sous air statique pendant au moins 30 min à une température comprise, de préférence, entre 300 et 700°C.

La présente invention a également pour objet un procédé de production d'un nitrile à partir d'un alcool en présence d'un catalyseur, caractérisé en ce qu'il comprend une étape d'ammoxydation d'un alcool de formule (II) suivante :

CH₂=C(R¹)-CH₂-OH (II)

dans laquelle R¹ représente un atome d'hydrogène ou un radical méthyle,
pour conduire à un nitrile de formule (III) suivante :

CH₂=C(R¹)-C≡N (III)

dans laquelle R¹ a la même signification que dans la formule (II) ci-dessus,
ladite réaction étant réalisée en phase gaz, ladite phase gaz comprenant au moins de l'ammoniac et de l'oxygène, et en présence d'un catalyseur solide choisi parmi les composés de formule (I) suivante :

SbₓFe₁O_{y} (I)

dans laquelle x varie de 0,4 à 1 inclusivement, et y varie de 1,6 à 4 inclusivement.

Le procédé conforme à l'invention pourrait permettre de se passer des ressources fossiles dans le cas où les matières premières (alcools de formule (II) et, en particulier, l'alcool allylique) seraient issues de la biomasse. Il est simple à mettre en oeuvre (une seule étape) et très sélectif. Il conduit aux nitriles de formule (III), en particulier à l'acrylonitrile (R₁ = H) avec des rendements supérieurs à 80 %. La séparation des co-produits de la réaction peut être réalisée selon des techniques connues de l'homme de l'art et ces co-produits peuvent également être valorisés car il s'agit d'intermédiaires d'intérêt dans l'industrie chimique.

Lorsque R¹ représente un atome d'hydrogène, alors l'alcool de formule (II) est l'alcool allylique et le nitrile de formule (III), l'acrylonitrile.

Lorsque R¹ est un radical méthyle, alors l'alcool de formule (II) est l'alcool méthallylique et le nitrile de formule (III) est le méthacrylonitrile.

Selon une forme de réalisation préférée de l'invention, R₁ représente un atome d'hydrogène. Ainsi, selon cette forme de réalisation préférée, le procédé conforme à l'invention comprend une étape d'ammoxydation de l'alcool allylique en acrylonitrile.

Le catalyseur est de préférence choisi parmi les composés de formule (I) dans lesquels x varie de 0,5 à 0,8 inclusivement. Un catalyseur de formule (I) particulièrement préféré selon l'invention est choisi parmi les composés dans lesquels x = 0,6.

La réaction d'ammoxydation est de préférence réalisée à une température supérieure ou égale à 400°C environ, et encore plus préférentiellement à une température variant de 350 à 450°C environ inclusivement.

Selon une forme de réalisation préférée du procédé conforme à l'invention, la réaction d'ammoxydation est conduite à pression atmosphérique.

Le temps de contact, défini comme étant le rapport entre le volume de catalyseur (en mL) et le débit volumique total de gaz injecté dans le réacteur (en mL/s), calculé à la température et à la pression de la réaction, varie de préférence de 0,05 à 2 s environ, et encore plus préférentiellement de 0,05 à 0,5 s environ.

Au sein de la phase gaz, le ratio molaire alcool de formule (II)/ammoniac peut varier de 1/1 à 1/4 environ. Selon une forme de réalisation préférée de l'invention, la réaction d'ammoxydation est conduite en utilisant une phase gaz dans laquelle le ratio molaire alcool de formule (II)/ammoniac est égal à 1/3 environ.

Au sein de la phase gaz, le ratio molaire alcool de formule (II)/oxygène peut varier de 1/1,5 à 1/5 environ. Selon une forme de réalisation préférée de l'invention, la réaction d'ammoxydation est conduite en utilisant une phase gaz dans laquelle le ratio molaire alcool de formule (II)/oxygène est égal à 1/3,5 environ.

Selon une forme de réalisation particulièrement préférée de l'invention, la réaction d'ammoxydation est conduite en utilisant une phase gaz dans laquelle le ratio molaire alcool de formule (II)/oxygène/ammoniac est de 1/3,5/3 environ.

Selon une forme de réalisation particulière du procédé conforme à l'invention, et bien que cela ne soit en aucun cas nécessaire au bon déroulement de la réaction d'ammoxydation, le catalyseur de formule (I) peut être supporté par un support solide poreux. Dans ce cas, le support solide poreux peut être choisi parmi les supports à base de silice, notamment sous forme de gel de silice (type CARiACT ®), de silice mésostructurée (comme par exemple la silice mésostructurée de type SBA-15), ainsi que parmi les supports à base d'oxydes de silice mixtes tels que par exemple SiO₂-TiO₂, SiO₂-ZrO₂; et les supports en carbure de silicium (SiC), etc...

Un tel support solide poreux présente de préférence une porosité moyenne comprise entre 0,1 cm³/g et 2,0 cm³/g inclusivement, et encore plus préférentiellement entre 0,5 cm³/g et 1,5 cm³/g inclusivement.

Lorsque la réaction est terminée, la séparation des co-produits de la réaction peut être réalisée par toutes techniques appropriées connues de l'homme de l'art, par exemple par distillation.

La présente invention est illustrée par les exemples de réalisation suivants, auxquels elle n'est cependant pas limitée.

### EXEMPLES

Dans les exemples qui suivent, les matières premières suivantes ont été utilisées :
- Acide oxalique à 97% (Fluka),
- Nitrate de fer nona-hydraté (Sigma Aldrich),
- Oxyde d'antimoine (III) (Sigma Aldrich),
- Alcool allylique à 99 % (Fluka),
- Ammoniac (Praxair),
- Oxygène (Alphagaz).

A partir de ces matières premières, des catalyseurs de formule (I) avec x = 0,4 à 1 ont été préparés. La valeur de y pour chacun de ces catalyseurs est déterminée par le respect de l'électro-neutralité et/ou des valences des éléments. Elle n'a pas été mesurée expérimentalement.

La synthèse de l'acrylonitrile a été réalisée en phase gaz dans un réacteur tubulaire à lit fixe de diamètre 15 mm et de longueur 120 mm. La température du réacteur a été régulée de manière précise et contrôlée par un thermocouple.

### EXEMPLE 1

### Synthèse d'un catalyseur de formule (I) avec x = 0,6

Une solution à 0,05 M a été préparée en dissolvant 2,21 g d'acide oxalique dans 500 mL d'eau à 80°C sous agitation. Une fois la dissolution achevée, 140,97 g de nitrate de fer nona-hydraté ont été ajoutés dans la solution d'acide oxalique en maintenant la température à 80°C. Après complète dissolution du nitrate de fer nona-hydraté, 30,51 g d'oxyde d'antimoine (III) ont été ajoutés. La solution résultante a été laissée évaporée en maintenant la température à 80°C, sous agitation, jusqu'à obtention d'une solution visqueuse qui a alors été séchée à l'étuve à 120°C pendant 72 heures. Après séchage, la produit obtenu a été pressé sous forme de pastilles qui ont ensuite été broyées pour obtenir un produit pulvérulent composé de particules ayant une dimension comprise entre 250 et 630 µm. Ces particules ont alors été calcinées sous air statique depuis la température ambiante jusqu'à 500°C en respectant une rampe de montée en température de 1°C/min puis une phase de maintien à 500°C pendant 8 heures. On a ensuite laissé le catalyseur dans le four jusqu'à ce que la température revienne à 50°C. On a obtenu un catalyseur présentant un ratio Sb/Fe de 0,6 (soit x = 0,6).

### EXEMPLE 2

### Synthèse d'un catalyseur de formule (I) avec x = 0,8

Un catalyseur de formule (I) dans lequel x = 0,8 a été préparé selon un mode opératoire identique à celui de l'Exemple 1 ci-dessus mais en utilisant 2,21 g d'acide oxalique, 27,7 g de nitrate de fer nona-hydraté et 8 g d'oxyde d'antimoine (III).

### EXEMPLE 3

### Synthèse d'un catalyseur de formule (I) avec x = 0,4

Un catalyseur de formule (I) dans lequel x = 0,4 a été préparé selon un mode opératoire identique à celui de l'Exemple 1 ci-dessus mais en utilisant 2,21 g d'acide oxalique, 87,3 g de nitrate de fer nona-hydraté et 12,6 g d'oxyde d'antimoine (III).

### EXEMPLE 4

### Synthèse d'un catalyseur de formule (I) avec x = 1,0

Un catalyseur de formule (I) dans lequel x = 1,0 a été préparé selon un mode opératoire identique à celui de l'Exemple 1 ci-dessus mais en utilisant 2,21 g d'acide oxalique, 22,2 g de nitrate de fer nona-hydraté et 8,0 g d'oxyde d'antimoine (III).

### EXEMPLE 5

### Synthèse de l'acrylonitrile à partir de l'alcool allylique

5 g du catalyseur préparé selon l'Exemple 1 ont été placés dans un réacteur à lit fixe. La réaction a été réalisée avec une solution aqueuse d'alcool allylique à 7,2 % en masse. Le réacteur a été chauffé à 400°C puis alimenté en réactifs (alcool allylique/O₂/NH₃), sous pression atmosphérique. Le temps de contact des réactifs avec le catalyseur était de l'ordre de 0,1 s et le temps de réaction de 5 heures.

Les produits issus de la réaction ont été analysés après piégeage en sortie de réacteur dans un barboteur maintenu à basse température (- 4°C). Le liquide obtenu a ensuite été analysé sur une chromatographie à phase gazeuse équipée d'un détecteur à ionisation de flamme.

Les conditions opératoires utilisées sont résumées dans le Tableau I ci-dessous :

**TABLEAU I**

| | **Témoin (*)** | **Essai 1** | **Essai 2** | **Essai 3** | **Essai 4** |
|---|---|---|---|---|---|
| Ratio molaire alcool allylique/O₂/NH₃ | 1/1,6/0,4 | 1/1,6/0,4 | 1/3,5/0,8 | 1/3,5/1,5 | 1/3,5/3 |
| Conversion de l'alcool allylique | 14 % | 87 % | 95 % | 99 % | 99 % |
| % Acrylonitrile | 0 | 17 | 38 | 52 | 63 |
| % Acroléine | 24 | 52 | 43 | 26 | 3 |
| % Acétaldéhyde | 16 | 5 | 4 | 1 | 1 |
| % Propionaldéhyde | 21 | 5 | 4 | 0 | <1 |
| % Acétonitrile | 0 | 1 | 2 | 2 | 1 |

| | | | | | |
|---|---|---|---|---|---|
| (*) : Procédé sans catalyseur : non conforme à l'invention | | | | | |

Ces résultats démontrent tout d'abord que la présence d'un catalyseur conforme à la présente invention est nécessaire pour réaliser la réaction d'ammoxydation de l'alcool allylique en acrylonitrile en présence d'ammoniac et d'oxygène. Ils montrent par ailleurs, que la présence d'un catalyseur conforme à la présente invention augmente, à conditions opératoires identiques, la conversion de l'alcool allylique (de 14 à 87 %) et augmente également la sélectivité en acrylonitrile et en acroléine, de 0 % et 24 % à 17 % et 52 %, respectivement. De plus, selon une forme de réalisation préférée du procédé conforme à l'invention, on voit que l'augmentation du rapport molaire NH₃/alcool allylique permet d'améliorer encore la sélectivité en acrylonitrile ainsi que la conversion de l'alcool allylique. Dans les conditions optimales de réalisation de cet exemple, la conversion de l'alcool allylique est de 99 % avec une sélectivité en acrylonitrile de 63 %.

### EXEMPLE 6

### Synthèse de l'acrylonitrile à partir de l'alcool allylique

Dans cet exemple, on a réalisé la réaction d'ammoxydation de l'alcool allylique selon le procédé détaillé à l'Exemple 5 ci-dessus dans des conditions opératoires permettant la conversion totale de l'alcool allylique, en utilisant le catalyseur préparé selon l'Exemple 1, à une température de 400 ou 450°C, et en utilisant différents rapports molaires alcool allylique / NH₃.

Le temps de réaction était de 5 heures.

Comme pour l'Exemple 5, les produits issus de la réaction ont été analysés après piégeage en sortie de réacteur dans un barboteur maintenu à basse température (-4°C). Le liquide obtenu, est ensuite analysé sur une chromatographie à phase gazeuse équipée d'un détecteur à ionisation de flamme.

Les conditions opératoires utilisées sont résumées dans les Tableaux II et III ci-dessous :

**TABLEAU II**

| | **Essai 5** | **Essai 6** | **Essai 7** |
|---|---|---|---|
| Température de réaction | 400°C | 450°C | 450°C |
| Temps de contact (s) | 0,1 | 0,1 | 0,16 |
| Ratio molaire alcool allylique/O₂/NH₃ | 1/3,5/2 | 1/3,5/1 | 1/3,5/2 |
| Conversion de l'alcool allylique | 100 % | 100 % | 100 % |
| % Acrylonitrile | 67 | 51 | 76 |
| % Acroléine | 11 | 24 | 9 |
| % Acétaldéhyde | 1 | 1 | 1 |
| % Propionaldéhyde | 1 | 1 | 1 |
| % Acétonitrile | 4 | 2 | 3 |
| Balance carbone | 97% | 101 % | 101 % |

**TABLEAU III**

| | **Essai 8** | **Essai 9** | **Essai 10** |
|---|---|---|---|
| Température de réaction | 450°C | 400°C | 400°C |
| Temps de contact (s) | 0,1 | 0,16 | 0,16 |
| Ratio molaire alcool allylique/O₂/NH₃ | 1/3,5/3 | 1/3,5/1 | 1/3,5/3 |
| Conversion de l'alcool allylique | 100 % | 100 % | 100 % |
| % Acrylonitrile | 83 | 50 | 76 |
| % Acroléine | 4 | 27 | 1 |
| % Acétaldéhyde | <1 | 2 | 1 |
| % Propionaldéhyde | 1 | 1 | 1 |
| % Acétonitrile | 3 | 3 | 5 |
| Balance carbone | 92% | 93% | 85 % |

Ces essais montrent que les meilleurs résultats en termes de sélectivité vis-à-vis de la formation d'acrylonitrile sont obtenus avec l'Essai 8 réalisé à 450°C avec un temps de contact de 0,1 s et un rapport molaire alcool allylique/NH₃ de 1/3. On obtient alors un rendement de 83% en acrylonitrile à conversion totale d'alcool allylique.

## Revendications

1. Utilisation d'un composé de formule (I) suivante :
SbₓFe₁O_{y} (I)
dans laquelle x varie de 0,4 à 1 inclusivement et y varie de 1,6 à 4 inclusivement, à titre de catalyseur pour catalyser la réaction d'ammoxydation d'un alcool de formule (II) suivante CH₂=C(R¹)-CH₂-OH (II) dans laquelle R¹ représente un atome d'hydrogène ou un radical méthyle en un nitrile de formule (III) suivante CH₂=C(R¹)-C≡N (III) dans laquelle R¹ a la même signification que dans la formule (II) ci-dessus, ladite réaction étant réalisée en phase gaz, ladite phase gaz comprenant au moins de l'oxygène et de l'ammoniac.

2. Procédé de production d'un nitrile à partir d'un alcool en présence d'un catalyseur, **caractérisé en ce qu'**il comprend une étape d'ammoxydation d'un alcool de formule (II) suivante :
CH₂=C(R¹)-CH₂-OH (II)
dans laquelle R¹ représente un atome d'hydrogène ou un radical méthyle,
pour conduire à un nitrile de formule (III) suivante :
CH₂=C(R¹)-C≡N (III)
dans laquelle R¹ a la même signification que dans la formule (II) ci-dessus,
ladite réaction étant réalisée en phase gaz, ladite phase gaz comprenant au moins de l'ammoniac et de l'oxygène, et en présence d'un catalyseur solide choisi parmi les composés de formule (I) suivante :
SbₓFe₁O_{y} (I)
dans laquelle x varie de 0,4 à 1 inclusivement et y varie de de 1,6 à 4 inclusivement.

3. Procédé selon la revendication 2, **caractérisé en ce que** R₁ représente un atome d'hydrogène et **en ce qu'**il comprend une étape d'ammoxydation de l'alcool allylique en acrylonitrile.

4. Procédé selon la revendication 2 ou 3, **caractérisé en ce que** le catalyseur est choisi parmi les composés dans lesquels x varie de 0,5 à 0,8 inclusivement.

5. Procédé selon l'une quelconque des revendications 2 à 4, **caractérisé en ce que** le composé de formule (I) est choisi parmi les composés dans lesquels x = 0,6.

6. Procédé selon l'une quelconque des revendications 2 à 5, **caractérisé en ce que** la réaction d'ammoxydation est réalisée à une température variant de 350 à 450°C.

7. Procédé selon l'une quelconque des revendications 2 à 6, **caractérisé en ce que** le rapport entre le volume de catalyseur et le débit volumique total de gaz injecté dans le réacteur, calculé à la température et à la pression de la réaction, varie de 0,05 à 2 s.

8. Procédé selon l'une quelconque des revendications 2 à 7, **caractérisé en ce qu'**au sein de la phase gaz, le ratio molaire alcool de formule (II)/ammoniac varie de 1/1 à 1/4.

9. Procédé selon l'une quelconque des revendications 2 à 8, **caractérisé en ce qu'**au sein de la phase gaz, le ratio molaire alcool de formule (II)/oxygène varie de 1/1,5 à 1/5.

10. Procédé selon l'une quelconque des revendications 2 à 9, **caractérisé en ce que** la réaction d'ammoxydation est conduite en utilisant une phase gaz dans laquelle le ratio molaire alcool de formule (II)/oxygène/ammoniac est de 1/3,5/3.

11. Procédé selon l'une quelconque des revendications 2 à 10, **caractérisé en ce que** le catalyseur de formule (I) est supporté par un support solide poreux.

## Patentansprüche

1. Verwendung einer Verbindung mit der folgenden Formel (I):
SbₓFe₁O_{y} (I)
wobei x von 0,4 bis einschließlich 1 variiert und y von 1,6 bis einschließlich 4 variiert, als Katalysator, um die Ammonoxidationsreaktion eines Alkohols mit der folgenden Formel (II) zu katalysieren: CH₂=C(R¹)-CH₂-OH (II), wobei R¹ ein Wasserstoffatom oder ein Methylradikal in einem Nitril der folgenden Formel darstellt: CH₂=C(R¹)-C≡C (III), wobei R¹ die gleiche Bedeutung wie in der Formel (II) unten aufweist, wobei die Reaktion in der Gasphase durchgeführt wird, wobei die Gasphase mindestens Sauerstoff und Ammoniak umfasst.

2. Verfahren zur Herstellung eines Nitrils ausgehend von einem Alkohol in Anwesenheit eines Katalysators, **dadurch gekennzeichnet, dass** es einen Schritt des Ammoxidierens eines Alkohols mit der folgenden Formel (II) umfasst:
CH₂=C(R¹)-CH₂-OH (II)
wobei R¹ ein Wasserstoffatom oder ein Methylradikal umfasst, um zu einem Nitril der folgenden Formel (III) zu gelangen:
CH₂=C(R¹)-C≡N (III);
wobei R¹ die gleiche Bedeutung wie in der Formel (II) unten aufweist,
wobei die Reaktion in der Gasphase durchgeführt wird, wobei die Gasphase mindestens Ammoniak und Sauerstoff umfasst, und in Anwesenheit eines festen Katalysators, ausgewählt aus den Verbindungen mit der folgenden Formel (I):
SbₓFe¹O_{y} (I)
wobei x von 0,4 bis einschließlich 1 variiert und y von 1,6 bis einschließlich 4 variiert.

3. Verfahren nach Anspruch 2, **dadurch gekennzeichnet, dass** R₁ ein Wasserstoffatom darstellt und dadurch, dass es einen Schritt des Ammoxydierens des Allylalkohols zu Acrylonitril umfasst.

4. Verfahren nach Anspruch 2 oder 3, **dadurch gekennzeichnet, dass** der Katalysator ausgewählt ist aus den Verbindungen, in denen x von 0,5 bis einschließlich 0,8 variiert.

5. Verfahren nach einem beliebigen der Ansprüche 2 bis 4, **dadurch gekennzeichnet, dass** die Verbindung der Formel (I) ausgewählt ist aus den Verbindungen, in denen x = 0,6.

6. Verfahren nach einem beliebigen der Ansprüche 2 bis 5, **dadurch gekennzeichnet, dass** die Ammoxidationsreaktion bei einer Temperatur durchgeführt wird, die von 350 bis 450 °C variiert.

7. Verfahren nach einem beliebigen der Ansprüche 2 bis 6, **dadurch gekennzeichnet, dass** das Verhältnis zwischen dem Katalysatorvolumen und dem gesamten Volumendurchsatz von Gas, das in den Reaktor injiziert ist, berechnet bei der Temperatur und bei dem Druck der Reaktion, von 0,05 bis 2s variiert.

8. Verfahren nach einem der vorhergehenden Ansprüche 2 bis 7, **dadurch gekennzeichnet, dass** innerhalb der Gasphase das Molverhältnis Alkohol mit der Formel (II)/Ammoniak von 1/1 bis 1/4 variiert.

9. Verfahren nach einem beliebigen der Ansprüche 2 bis 8, **dadurch gekennzeichnet, dass** innerhalb der Gasphase das Molverhältnis Alkohol mit der Formel (II)/Sauerstoff von 1/1,5 bis 1/5 variiert.

10. Verfahren nach einem beliebigen der Ansprüche 2 bis 9, **dadurch gekennzeichnet, dass** die Amoxidationsreaktion unter Verwendung einer Gasphase durchgeführt wird, in der das Molverhältnis Alkohol mit der Formel (II)/Sauerstoff 1/3, 5/3 ist.

11. Verfahren nach einem beliebigen der Ansprüche 2 bis 10, **dadurch gekennzeichnet, dass** der Katalysator mit der Formel (I) durch einen festen porösen Träger getragen wird.

## Claims

1. Use of a compound of following formula (I):
SbₓFe₁O_{y} (I)
in which x varies from 0.4 to 1 inclusive and y varies from 1.6 to 4 inclusive, as catalyst for catalysing the ammoxidation reaction of an alcohol of following formula (II) CH₂=C(R¹)-CH₂-OH (II), in which R¹ represents a hydrogen atom or a methyl radical, to give a nitrile of following formula (III) CH₂=C(R¹)-C≡N (III), in which R¹ has the same meaning as in the above formula (II), the said reaction being carried out in the gas phase, the said gas phase comprising at least oxygen and ammonia.

2. Process for the production of a nitrile from an alcohol in the presence of a catalyst, **characterized in that** it comprises a stage of ammoxidation of an alcohol of following formula (II):
CH₂=C(R¹)-CH₂-OH (II)
in which R¹ represents a hydrogen atom or a methyl radical,
to result in a nitrile of following formula (III):
CH₂=C(R¹)-C≡N (III)
in which R¹ has the same meaning as in the above formula (II),
the said reaction being carried out in the gas phase, the said gas phase comprising at least ammonia and oxygen, and in the presence of a solid catalyst chosen from the compounds of following formula (I):
SbₓFe₁O_{y} (I)
in which x varies from 0.4 to 1 inclusive and y varies from 1.6 to 4 inclusive.

3. Process according to Claim 2, **characterized in that** R¹ represents a hydrogen atom and **in that** it comprises a stage of ammoxidation of allyl alcohol to give acrylonitrile.

4. Process according to Claim 2 or 3, **characterized in that** the catalyst is chosen from the compounds in which x varies from 0.5 to 0.8 inclusive.

5. Process according to any one of Claims 2 to 4, **characterized in that** the compound of formula (I) is chosen from the compounds in which x = 0.6.

6. Process according to any one of Claims 2 to 5, **characterized in that** the ammoxidation reaction is carried out at a temperature varying from 350 to 450°C.

7. Process according to any one of Claims 2 to 6, **characterized in that** the ratio of the volume of catalyst to the total flow rate by volume of gas injected into the reactor, calculated at the temperature and at the pressure of the reaction, varies from 0.05 to 2 s.

8. Process according to any one of Claims 2 to 7, **characterized in that**, within the gas phase, the alcohol of formula (II)/ammonia molar ratio varies from 1/1 to 1/4.

9. Process according to any one of Claims 2 to 8, **characterized in that**, within the gas phase, the alcohol of formula (II)/oxygen molar ratio varies from 1/1.5 to 1/5.

10. Process according to any one of Claims 2 to 9, **characterized in that** the ammoxidation reaction is carried out using a gas phase in which the alcohol of formula (II)/oxygen/ammonia molar ratio is 1/3.5/3.

11. Process according to any one of Claims 2 to 10, **characterized in that** the catalyst of formula (I) is supported by a porous solid support.
